# EUROPEAN PATENT APPLICATION

(11) **EP 4 265 720 A1**
(43) Date of publication of application: **25.10.2023**
(21) Application number: 21907151.1
(22) Date of filing: 17.12.2021
(51) Int. Cl.: C12N 11/08, C12N 1/20, A61K 9/50, A61K 9/19, A61K 35/741, A23L 33/135, A61P 1/00, C12N 1/04, A61K 35/00

(54) **NON-STOP PRODUCTION PROCESS FOR IMPROVING FREEZE-DRYING SURVIVAL, HEAT TOLERANCE, SHELF STABILITY AND DIGESTIVE STABILITY OF PROBIOTICS USING SPONTANEOUS MATRIX-ENCAPSULATION TECHNIQUE**

(30) Priority: 18.12.2020 KR 20200178578
(71) Applicant: Ildong Pharmaceutical Co., Ltd., Seoul 06752 (KR)
(72) Inventor: KIM, Young-Hoo, Hwaseong-si Gyeonggi-do 18449 (KR); KIM, Hyeong-Eun, Hwaseong-si Gyeonggi-do 18449 (KR); HAN, Chi-Young, Hwaseong-si Gyeonggi-do 18449 (KR); SEO, Han Sol, Hwaseong-si Gyeonggi-do 18449 (KR); KIM, Tae-Yoon, Hwaseong-si Gyeonggi-do 18449 (KR); LEE, Sunghee, Hwaseong-si Gyeonggi-do 18449 (KR)
(74) Representative: Mewburn Ellis LLP
(86) International application number: PCT/KR2021/019274
(87) International publication number: WO 2022/131847

(57) **Abstract**

Disclosed is a process for non-stop production of encapsulated probiotics, in which an alginate hydrogel is spontaneously formed through a simple process of culturing probiotics in a medium containing an alginate, a salt that forms a hydrogel by binding to alginic acid, preferably an insoluble carbonate, and optionally an encapsulation enhancer, and probiotics are encapsulated by the alginate hydrogel, thereby not only greatly improving the probiotic encapsulation process but also remarkably improving the freeze-drying viability, heat tolerance, shelf stability, and *in-vivo* stability (acid resistance and bile resistance) of probiotics.

## Description

### BACKGROUND OF THE INVENTION

### Field of the Invention

The present invention relates to a process for non-stop production of encapsulated probiotics through a technique for spontaneously forming a matrix capsule protective film, and more particularly to a method of producing encapsulated probiotics in which probiotics are entrapped within an alginate hydrogel that is spontaneously formed by culturing probiotics in a medium containing an alginate, a salt that forms a hydrogel by binding to alginic acid, and, optionally, an encapsulation enhancer.

### Description of the Related Art

Probiotics are live microorganisms that have beneficial effects on health *in vivo.* Most probiotics known to date are lactic acid bacteria, and some include *Bacillus* and the like. The functions of lactic acid bacteria and probiotics have been studied ever since Russian scientist Elie Metchnikoff won the Nobel Prize for discovering that the reason Bulgarians enjoy longevity is due to the consumption of milk fermented with *Lactobacillus* (Mercenier A. et al., Curr. Pharm. Des. 9(2):175-191, 2003).

Representative effects of probiotics include antibacterial activity, antibiotic-related diarrhea alleviation, lactose intolerance reduction, anticancer activity, blood cholesterol reduction, inhibition of *Helicobacter pylori* in the stomach, irritable colitis relief, Crohn's disease relief, ulcerative colitis relief, immune function regulation, and the like. Probiotics may be classified into intestinal medicaments or lactic acid bacteria preparations, which are human medicaments, probiotics as feed additives, and lactic acid bacteria foods, which are a kind of health food. A lactic acid bacteria food is a product made into powder, granules, tablets, and capsules for stable and easy intake by culturing live bacteria such as *Lactobacillus, Lactococcus, Bifidobacterium,* etc. and mixing the same with food, and includes products other than lactic acid bacteria fermented foods, lactic acid bacteria fermented milk, and lactic acid bacteria beverages.

Lactic acid bacteria are bacteria that mainly produce lactic acid as a result of fermentation, among bacteria grown by fermentation. Lactic acid bacteria are one of the most important microbial groups in microbiological industries, and are principally used to produce fermented foods such as kimchi, yogurt, cheese, buttermilk, and sauerkraut, and are also widely used as functional probiotics that promote health.

Currently, lactic acid bacteria are classified into 12 genera (*Lactobacillus, Carnobacterium, Atopobium, Lactococcus, Pediococcus, Tetragenococcus, Leuconostoc, Weissella, Oenococcus, Enterococcus, Streptococcus,* and *Vagococcus*), and examples of lactic acid bacteria that are typically used include bacilli such as *Lactobacillus* spp. and cocci such as *Lactococcus* spp., *Streptococcus* spp., *Leuconostoc* spp., and *Pediococcus* spp. (Taejin Kang, BioWave, 2009, Vol 11, No 7, pp 1-20).

The process of producing lactic acid bacteria into food broadly includes culture of lactic acid bacteria, recovery of cells, freeze-drying, pulverization, commercialization, etc. In this procedure, lactic acid bacteria are exposed to various physical and chemical stresses. Specifically, during recovery of cells, lactic acid bacteria are affected by osmotic pressure in the concentration process, and during freeze-drying, lactic acid bacteria are affected both by temperature and osmotic pressure due to dehydration and the formation of ice crystals in the cytoplasm or the formation of ice crystals outside cells due to a drastic temperature change. Moreover, when exposed to high temperature and high pressure or hydrated by water in the air during pulverization and commercialization, lactic acid bacteria become unstable. When not only liquid products such as lactic acid bacteria fermented foods, lactic acid bacteria fermented milk, and lactic acid bacteria beverages stored for a short period of time, but also products in powder form for long-term storage are exposed to oxygen, a problem has been noted in that fatty acids constituting the cell membrane are oxidized and the viability is lowered (Korean Patent No. 10-1605516).

Meanwhile, in order for bacteria, including lactic acid bacteria, to be recognized as probiotics, they must survive gastric acid and bile acid, reach the small intestine, and proliferate and settle in the intestine, and have to exhibit useful effects in the intestinal tract and be non-toxic and non-pathogenic. However, various beneficial bacteria including lactic acid bacteria are exposed to an acidic environment in which the pH drops to 3 or less in the stomach and are affected by bile acids in the small intestine, so the viability thereof is greatly reduced.

Therefore, various methods of encapsulating bacteria have been developed to enhance stability in the process of freeze-drying these beneficial bacteria and to increase the viability of beneficial bacteria in the process of reaching the intestine in the body. For example, Korean Patent Application Publication No. 2003-0009268 discloses a method of producing microcapsules in which lactic acid bacteria are cultured and stirred with alginic acid and CaCO₃ to obtain a mixture of a microcapsule-forming composition and lactic acid bacteria, and the mixture of microcapsule-forming composition and lactic acid bacteria is sprayed with a coagulation solution containing a small amount of acetic acid (CH₃COOH) and CaCl₂, cured, and secondarily cured with CaCl₂.

However, this encapsulation process does not completely coat the surface of the cells, so heat tolerance, acid resistance and bile resistance of the strain are still not sufficiently improved, and moreover, there is a problem in that aseptic processing for probiotics is difficult because it is necessary to undertake multiple steps in which the typically cultured cells are recovered and then mixed with a coating composition with stirring. In particular, economic benefits are negated in industrial mass production, which is undesirable.

Accordingly, the present inventors have endeavored to develop a new encapsulation process that exhibits improved *in-vivo* stability of probiotics and is simplified compared to conventional probiotic encapsulation processes, and have ascertained that, when culturing probiotics in a medium containing alginate and carbonate at predetermined concentrations, freeze-drying viability, heat tolerance, and *in-vivo* stability (acid resistance and bile resistance) of probiotics may be remarkably improved, and short-term and long-term shelf stability thereof may also be greatly improved, thus culminating in the present invention.

### Citation List

### Patent Literature

(Patent Document 1) Korean Patent No. 10-1605516
(Patent Document 2) Korean Patent Application Publication No. 10-2003-0009268

### Non-Patent Literature

(Non-Patent Document 1) Taejin Kang, BioWave, 2009, Vol. 11, No. 7, pp 1-20
(Non-Patent Document 2) Mercenier A. et al., Curr. Pharm. Des. 9(2):175-191, 2003

### SUMMARY OF THE INVENTION

It is an object of the present invention to provide a method of producing encapsulated probiotics exhibiting the improved freeze-drying viability, heat tolerance, shelf stability, and *in-vivo* stability of lactic acid bacteria through a technique for spontaneously forming a matrix capsule protective film, which is very simple compared to conventional processes for producing encapsulated probiotics, and to provide the use of encapsulated probiotics produced through the above method.

In order to accomplish the above objects, the present invention provides a method of producing encapsulated probiotics in which probiotics are entrapped within an alginate hydrogel, including:
(a) spontaneously forming an alginate hydrogel by culturing probiotics in a medium containing an alginate and a salt that forms a hydrogel by binding to alginic acid; and
(b) recovering the probiotics encapsulated by the spontaneously formed alginate hydrogel.

In addition, the present invention provides encapsulated probiotics produced through the above method, and a composition, food, functional health food, cosmetic, and medicament including the same.

In addition, the present invention provides the use of the encapsulated probiotics of the present invention for the manufacture of a composition, food, functional health food, cosmetic, and/or medicament.

In addition, the present invention provides the use of the encapsulated probiotics of the present invention for the prevention or treatment of disease.

In addition, the present invention provides a method of alleviating, preventing or treating a disease including administering the encapsulated probiotics of the present invention.

### BRIEF DESCRIPTION OF THE DRAWINGS

The above and other objects, features, and other advantages of the present invention will be more clearly understood from the following detailed description taken in conjunction with the accompanying drawings, in which:

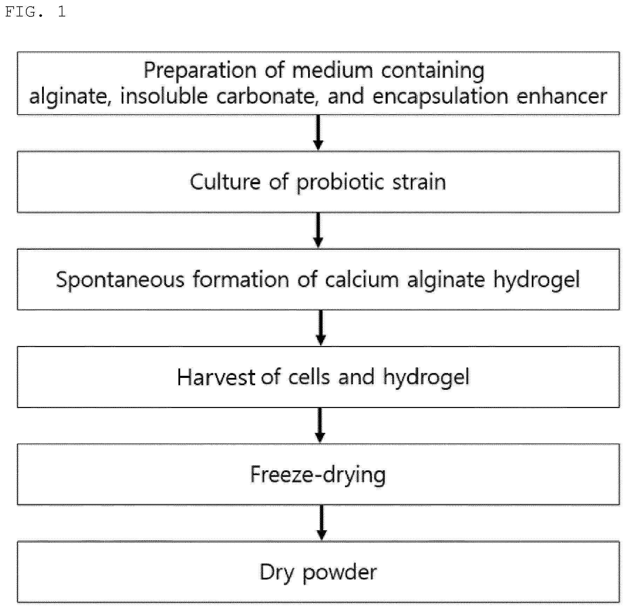

FIG. 1 shows a process of producing encapsulated probiotics according to a preferred embodiment of the present invention;
FIG. 2 shows the particle sizes of probiotic capsules produced through a conventional alginate encapsulation process and the encapsulation process of the present invention; and
FIG. 3 shows SEM images of dry cells (A) on which encapsulation is not carried out, dry cells (B) to which only an encapsulant (alginate) is applied, and dry cells (C) to which both an encapsulant (alginate) and an encapsulation enhancer (starch) are applied, respectively (a scale increment of 1 um).

### DETAILED DESCRIPTION OF THE INVENTION

Unless otherwise defined, all technical and scientific terms used herein have the same meanings as those typically understood by those skilled in the art to which the present invention belongs. Generally, the nomenclature used herein is well known in the art and is typical.

In the present invention, it is confirmed that, when culturing probiotics in a medium containing both an alginate and a salt that forms a hydrogel by binding to alginic acid, a divalent cation is liberated from the salt that forms a hydrogel by binding to alginic acid due to the action of the acid generated by the proliferation of probiotics, and binds to alginic acid in the medium to thus spontaneously form an alginic acid/divalent cation hydrogel, after which the spontaneously formed alginic acid/divalent cation hydrogel is recovered along with the cells, thereby producing capsules in which probiotics are encapsulated through an extremely simple process compared to the conventional process.

For example, when the salt that forms a hydrogel by binding to alginic acid is calcium carbonate, calcium is liberated from calcium carbonate by the acid generated during culture of probiotics, and the liberated calcium binds to alginate to thus spontaneously form a calcium alginate hydrogel.

Accordingly, the present invention pertains to a method of producing encapsulated probiotics, including:
(a) spontaneously forming an alginate hydrogel by culturing probiotics in a medium containing an alginate and a salt that forms a hydrogel by binding to alginic acid; and
(b) recovering the probiotics encapsulated by the spontaneously formed alginate hydrogel.

As used herein, the term "encapsulation" refers to coating a raw material with an encapsulant or entrapping the raw material inside the encapsulant. For example, encapsulation of a reservoir type in which probiotics are present inside a capsule shell composed of an encapsulant or a matrix type in which probiotics are entrapped within a matrix composed of an encapsulant may be performed (Zuidam and Nedovic, (2010) Encapsulation Technologies for Active Food Ingredients and Food Processing, Springer), but the present invention is not limited thereto. In the present invention, the encapsulation is capable of stabilizing probiotics or protecting probiotics from the external environment and the *in-vivo* environment.

As used herein, the term "encapsulated probiotics" refers to probiotics in the form of being coated with various encapsulants such as carbohydrates, syrups, gums, hydrogels, etc., or in the form of being entrapped within a matrix. In the present invention, the term "encapsulated probiotics" may be used interchangeably with "probiotic capsules", and may have the same meaning.

In the present invention, the encapsulated probiotics may be of a reservoir type in which probiotics are present inside a capsule shell composed of an encapsulant or a matrix type in which probiotics are entrapped within a matrix composed of an encapsulant, but the present invention is not limited thereto.

In the present invention, the probiotics of the encapsulated probiotics are entrapped within the alginate hydrogel.

As used herein, the term "entrapping" or "entrapped" means that probiotics are encapsulated within the space or pores included within the structure of the alginate hydrogel of the present invention. In addition to the probiotics, a culture composition such as a medium, water, nutrients, and the like, may be encapsulated within the space or pores.

In the present invention, the alginate preferably has solubility in water of 0.1 g/L or more, and more preferably 0.1 g/L to 10 g/L, and is preferably selected from the group consisting of sodium alginate, potassium alginate, magnesium alginate, calcium alginate, lithium alginate, and ammonium alginate, but is not limited thereto, and any alginate meeting the purpose of the present invention may be used without limitation.

In the present invention, the amount of the alginate that is contained in the medium is 0.1 to 40 g/L, preferably 0.2 to 15 g/L, more preferably 0.5 to 2 g/L, and most preferably 0.8 to 1.2 g/L, but is not limited thereto.

In addition, the salt that forms a hydrogel by binding to alginic acid according to the present invention may be a divalent cation salt having solubility in water of 0.1 g/L or less, and preferably 0.00001 g/L to 0.1 g/L, and is preferably a basic salt containing a divalent cation. For example, the salt that forms a hydrogel by binding to alginic acid may be a carbonate or nitrate containing a divalent cation.

For example, the carbonate containing the divalent cation may be at least one selected from the group consisting of calcium carbonate, barium carbonate, manganese carbonate, copper carbonate, zinc carbonate, lead carbonate, cadmium carbonate, cobalt carbonate, nickel carbonate, and strontium carbonate, but is not limited thereto.

In another example, the nitrate containing the divalent cation may be at least one selected from the group consisting of calcium nitrate, barium nitrate, manganese nitrate, copper nitrate, zinc nitrate, lead nitrate, cadmium nitrate, cobalt nitrate, nickel nitrate, and strontium nitrate, but is not limited thereto.

In the present invention, the amount of the salt that forms a hydrogel by binding to alginic acid is 0.5 to 10 g/L, preferably 1 to 9 g/L, more preferably 1.5 to 8 g/L, and most preferably 2 to 7 g/L, but is not limited thereto.

In the present invention, the salt that forms a hydrogel by binding to alginic acid may be configured such that the cation is dissociated by the acid generated by culturing the probiotics.

In the present invention, the acid generated by culturing the probiotics is an organic acid produced during metabolism of probiotics. Examples of the acid generated by culturing the probiotics may include, but are not limited to, lactic acid, acetic acid, citric acid, malic acid, succinic acid, etc.

In the present invention, in step (a), the acid generated by culturing the probiotics may cause dissociation of the cation from the salt that forms a hydrogel by binding to alginic acid.

In the present invention, in step (a), the alginate hydrogel may be formed by coupling alginic acid with the cation dissociated from the salt that forms a hydrogel by binding to alginic acid.

In the present invention, in step (a), the probiotics may be proliferated by culture.

In the present invention, in step (a), all or part of the cultured probiotics may be entrapped within the spontaneously formed alginate hydrogel.

Moreover, in the present invention, the medium in step (a) may further contain an encapsulation enhancer.

The encapsulation enhancer is used to increase the robustness of the alginate hydrogel capsule, and is insoluble. For example, the encapsulation enhancer may be at least one selected from the group consisting of starch, crystalline cellulose, chitosan, carboxymethyl cellulose (CMC), and skim milk powder, but is not limited thereto. Taking starch as an example of the encapsulation enhancer, when the medium additionally contains starch, the hydrogel resulting from binding of the salt that forms a hydrogel by binding to alginic acid and starch are mixed with probiotics, thereby producing probiotic capsules having a more robust structure.

In the present invention, the amount of the encapsulation enhancer that is contained in the medium is 1 to 20 g/L, preferably 2 to 10 g/L, more preferably 3 to 5 g/L, and most preferably 5 g/L.

In an embodiment of the present invention, it is confirmed that the probiotic capsules produced through the encapsulation method of the present invention without the use of a cryoprotectant exhibit vastly superior freeze-drying viability, heat tolerance, survival rate in a simulated gastrointestinal solution, and long-term/short-term shelf stability compared to a formulation produced using calcium carbonate and a cryoprotectant.

In the present invention, the medium may contain a cryoprotectant as in a conventional medium, but excellent freeze-drying viability, heat tolerance, survival rate in the simulated gastrointestinal solution, and long-term/short-term shelf stability may be exhibited even without the use of a cryoprotectant. It is preferable that the medium not contain a cryoprotectant.

Moreover, in the present invention, it is confirmed that the pH of the culture broth is prevented from excessively decreasing in the course of dissociation of a divalent cation salt, for example, calcium carbonate, into calcium ions by the acid secreted by probiotics during culture of probiotics, so the probiotics may be efficiently cultured without the need to additionally adjust the pH in the culture step.

Accordingly, in the present invention, during the culture of probiotics in a medium containing an alginate and a salt that forms a hydrogel by binding to alginic acid, an additional pH adjustment step or process is not performed, or a minimal pH adjustment step or process is performed compared to the conventional culture process.

In the corresponding process, the inoculation concentration and culture time of the probiotic strain may be varied as appropriate depending on the type of strain, as will be apparent to those skilled in the art. Here, the initial concentration of the strain after inoculation may be 1×10⁴ to 5×10⁹ CFU/mL, and preferably 5×10⁵ to 4×10⁸ CFU/mL, but is not limited thereto. Also, the culture time of the probiotic strain is preferably 4 hours or more, and more preferably 6 hours or more so that the salt that forms a hydrogel by binding to alginic acid, such as carbonate, may be sufficiently dissociated, but the present invention is not limited thereto.

In the present invention, step (b) may be performed by recovering all or part of the cultured probiotics and the spontaneously formed alginate hydrogel in step (a).

In the present invention, the encapsulated probiotics recovered in step (b) may be configured to be naturally entrapped within the spontaneously formed alginate hydrogel in step (a).

In the present invention, during the recovery in step (b), some probiotics not entrapped within the spontaneously formed alginate hydrogel in step (a) may be additionally entrapped within the alginate hydrogel.

In the present invention, in step (b), when the encapsulation enhancer is additionally contained in the medium of step (a), the alginate hydrogel, the encapsulation enhancer, and the probiotics may be recovered.

In the present invention, step (b) may include cooling the result of culture in step (a). In the present invention, the cooling may be performed at a temperature equal to or lower than the culture temperature, preferably at 37°C or less, and more preferably at about 20°C or less, and in an embodiment of the present invention, the result of culture is cooled to about 20°C, but the present invention is not limited thereto.

In the present invention, step (b) may include performing centrifugation.

In the present invention, step (b) may be performed by simultaneously recovering the alginate hydrogel and the probiotics.

In the present invention, in step (b), the culture broth from step (a) is stirred or mixed simultaneously with or before recovery. For example, the alginate hydrogel and the probiotics, or the alginate hydrogel, the encapsulation enhancer, and the probiotics may be recovered and mixed at the same time in a tank that recovers cells and is provided with a stirrer.

In the present invention, some probiotics that are not naturally entrapped within the alginate hydrogel formed in step (a) may be additionally entrapped through mixing.

In the present invention, (c) freeze-drying the recovered encapsulated probiotics may be further performed.

In the present invention, when (c) freeze-drying the recovered encapsulated probiotics is further performed, stirring may be conducted in the course of dispensing the recovered material in a freeze-dryer in step (c), and natural mixing may be carried out.

In the present invention, stirring or mixing may be additionally performed after step (b) and before step (c). Specifically, the alginate hydrogel and the probiotics may be mixed simultaneously with recovery, or may be mixed additionally after recovery.

In the present invention, the probiotics may be selected from the group consisting of *Lactobacillus* sp., *Bifidobacterium* sp., *Streptococcus* sp., *Lactococcus* sp., *Enterococcus* sp., *Leuconostoc* sp., *Pediococcus* sp., and *Weissella* sp. Since the technical feature of the present invention is to verify that superior alginate hydrogel coating during strain culture is possible as a method for improving the production, distribution, and *in-vivo* stability of beneficial bacteria when ingested by animals, including humans, it will be apparent to those skilled in the art that the present invention is applicable to beneficial bacteria other than the bacteria described above.

In the present invention, the probiotics may be, for example, at least one selected from among the strains shown in Table 1 below, but are not limited thereto.

**[Table 1]**

| **Genus** | **Species** | **Genus** | **Species** |
|---|---|---|---|
| *Lactobacillus* | *Lactobacillus acidophilus* | *Bifidobacterium* | *Bifidobacterium adolescentis* |
| | *Lactobacillus brevis* | | *Bifidobacterium animalis* subsp. *animalis* |
| | *Lactobacillus casei* | | *Bifidobacterium animalis* subsp. *lactis* |
| | *Lactobacillus crispatus* | | *Bifidobacterium bifidum* |
| | *Lactobacillus curvatus* | | *Bifidobacterium breve* |
| | *Lactobacillus delbrueckii* subsp. *bulgaricus* | | *Bifidobacterium dentium* |
| | *Lactobacillus delbrueckii* subsp. *delbrueckii* | | *Bifidobacterium longum* subsp. *infantis* |
| | *Lactobacillus fermentum* | | *Bifidobacterium longum* subsp. *longum* |
| | *Lactobacillus gasseri* | *Lactococcus* | *Lactococcus lactis* subsp. *cremoris* |
| | *Lactobacillus helveticus* | | *Lactococcus lactis* subsp. *lactis* |
| | *Lactobacillus jensenii* | *Streptococcus* | *Streptococcus diacetylactis* |
| | *Lactobacillus johnsonii* | | *Streptococcus salivarius* |
| | *Lactobacillus paracasei* | | *Streptococcus thermophilus* |
| | *Lactobacillus pentosus* | *Leuconostoc* | *Leuconostoc citreum* |
| | *Lactobacillus plantarum* | | *Leuconostoc kimchii* |
| | *Lactobacillus reuteri* | | *Leuconostoc mesenteroides* |
| | *Lactobacillus rhamnosus* | | *Leuconostoc paramesenteroides* |
| | *Lactobacillus sake!* | *Weissella* | *Weissella cibaria* |
| | *Lactobacillus salivarius* | | *Weissella confuse* |
| *Pediococcus* | *Pediococcus acidilactici* | | *Weissella koreensis* |
| | *Pediococcus halophilus* | *Enterococcus* | *Enterococcus faecium* |
| | *Pediococcus parvulus* | | *Enterococcus lactis* |
| | *Pediococcus pentosaceus* | *Saccharomyces* | *Saccharomyces boulardii* |

The genus or species name of the strain used in the specification of the present invention may be different from the genus name re-established by Zheng et al. (Int. J. Syst. Evol. Microbiol. 2020; 70), and the genus/species of the strain may be easily understood by those skilled in the art with reference to the description of the corresponding document.

Meanwhile, compared to the conventional technique for introducing a separate encapsulation step after completion of culture of probiotics in the probiotic encapsulation method, the process of the present invention is simplified as described above, which is advantageous in view of production economy as well as reducing concerns about contamination that may be caused by the introduction of a separate encapsulation step or improving convenience in quality control, and moreover, freeze-drying viability, heat tolerance, shelf stability, and *in-vivo* stability of the produced probiotics are remarkably improved compared to probiotic capsules produced through the conventional technique.

Accordingly, another aspect of the present invention pertains to encapsulated probiotics produced through the method of producing encapsulated probiotics according to the present invention.

As used herein, the terms "encapsulation" and "encapsulated probiotics" may be understood to have the same meaning as those described in the method of producing encapsulated probiotics above.

In the present invention, the encapsulated probiotics may be of a reservoir type in which probiotics are present inside a capsule shell composed of an encapsulant or a matrix type in which probiotics are entrapped within a matrix composed of an encapsulant, and are preferably of a matrix type (Zuidam and Nedovic, (2010) Encapsulation Technologies for Active Food Ingredients and Food Processing, Springer), but the present invention is not limited thereto.

In the present invention, the encapsulated probiotics may be configured such that the probiotics are entrapped within the alginate hydrogel.

In the present invention, the encapsulated probiotics may be used as a raw material, preferably a functional raw material, which is included in fermented milk, processed milk, fermented soy sauce, fermented kimchi, functional beverages, functional foods, general foods, medicaments, cosmetics, etc., and are preferably used as an active ingredient for functional health foods for oral administration.

Accordingly, still another aspect of the present invention pertains to a composition containing the encapsulated probiotics according to the present invention, and to a food, a functional health food, and a medicament including the same.

Yet another aspect of the present invention pertains to the use of the encapsulated probiotics of the present invention or the composition containing the same for the manufacture of a food and/or a functional health food.

As used herein, the term "food" refers to meat, sausages, bread, chocolate, candy, snacks, confectioneries, pizza, ramen, other noodles, gum, dairy products including ice cream, various soups, beverages, tea, drinks, alcoholic beverages, vitamin complexes, functional health foods, and health foods, and includes all foods in the ordinary sense.

The term "functional health food" has the same meaning as the term "food for special health use (FoSHU)", and refers to a food having high pharmaceutical and medical effects processed in order to efficiently show bioregulatory functions, in addition to nutritional supply. Here, "functional" means obtaining useful effects for health purposes, such as nutrient regulations or physiological actions with regard to the structure and function of the human body. The food of the present invention may be manufactured using a method commonly used in the art, and at the time of manufacture, raw materials and components commonly used in the art may be added. Moreover, the form of the food may not be limited, so long as it is a formulation recognized as a food, and the functional health food according to the present invention may take the form of a powder, granule, tablet, capsule, or beverage.

Health food is a food having an active effect on health maintenance or promotion beyond that of general food, and health supplement food is a food for health supplement purposes. In some cases, the terms "functional health food", "health food", and "health supplement food" are used interchangeably.

The food composition may further include a physiologically acceptable carrier, the type of which is not particularly limited, and any carrier commonly used in the art may be used.

Also, the composition may include additional ingredients that are commonly used in food compositions to improve smell, taste, visual appearance, and the like. For example, it may include vitamins A, C, D, E, B1, B2, B6, B12, niacin, biotin, folate, pantothenic acid, and the like. In addition, it may include minerals such as zinc (Zn), iron (Fe), calcium (Ca), chromium (Cr), magnesium (Mg), manganese (Mn), copper (Cu), chromium (Cr), and the like. In addition, it may include amino acids such as lysine, tryptophan, cysteine, valine, and the like.

Additionally, the composition may include food additives, such as preservatives (potassium sorbate, sodium benzoate, salicylic acid, sodium dehydroacetate, etc.), disinfectants (bleaching powder, high bleaching powder, sodium hypochlorite, etc.), antioxidants (butylhydroxyanisole (BHA), butylhydroxy toluene (BHT), etc.), colorants (tar pigment, etc.), color development agents (sodium nitrite, etc.), bleaches (sodium sulfite), seasonings (MSG, etc.), sweeteners (dulcin, cyclamate, saccharin, sodium, etc.), flavorings (vanillin, lactones, etc.), expansion agents (alum, D-potassium hydrogen tartrate, etc.), strengthening agents, emulsifiers, thickening agents (thickeners), film-forming agents, gum base agents, foam inhibitors, solvents, improving agents, and the like. The additive may be selected depending on the type of food, and may be used in an appropriate amount.

In the present invention, the food may further include a food supplement additive that is acceptable for human consumption, in addition to the encapsulated probiotics produced through the method of the present invention, and may be used along with other foods or food ingredients, and may be used appropriately according to a typical method. The amount of the active ingredient that is included therein may be appropriately determined according to the purpose of use thereof (prevention, health or therapeutic treatment).

The food containing the encapsulated probiotics according to the present invention may be used in the form of fermented milk, processed milk, fermented soy sauce, fermented kimchi, and general foods, but is not limited thereto.

Also, the functional health food containing the encapsulated probiotics according to the present invention preferably takes a form selected from the group consisting of a powder, granule, tablet, capsule, beverage, tea, fermented milk, processed milk, fermented soy sauce, fermented kimchi, and vitamin complex, but the present invention is not limited thereto.

In addition, the present invention pertains to a pharmaceutical composition and a medicament including the encapsulated probiotics according to the present invention.

Still yet another aspect of the present invention pertains to the use of the encapsulated probiotics of the present invention or the composition including the same for the manufacture of a pharmaceutical composition and/or a medicament.

The terms "prevention" and "treatment" when used in the present invention should be interpreted in the broadest sense. Here, "prevention" means preventing the progression of one or more of the clinical symptoms of a disease in a patient who may be exposed to or susceptible to the disease, but who has not yet experienced or revealed symptoms of the disease, and "treatment" means any action that arrests or reduces the development of a disease or one or more clinical symptoms thereof.

The pharmaceutical composition may further include suitable carriers, excipients, and diluents commonly used in pharmaceutical compositions, in addition to containing the encapsulated probiotics of the present invention as an active ingredient.

The carriers, excipients, and diluents that may be included in the composition include, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. When formulating the composition, it may be prepared using diluents or excipients such as fillers, extenders, binders, wetting agents, disintegrants, surfactants, etc., which are commonly used.

The pharmaceutical composition according to the present invention may be formulated and used in various dosage forms according to a typical method. Suitable dosage forms preferably include oral formulations such as tablets, pills, powders, granules, sugar-coated tablets, hard or soft capsules, solutions, suspensions, emulsions, injections, aerosols, and the like. Among these formulations, granules and powder pellets are most preferred. An oral medicament containing a *Platycodon grandiflorum* extract is exemplified by Yonggaksan. Other formulations include, but are not limited to, external preparations, suppositories, and sterile injection solutions.

The pharmaceutical composition according to the present invention may be formulated in a suitable dosage form using a pharmaceutically inert organic or inorganic carrier. Specifically, when the formulation is a tablet, a coated tablet, a sugar-coated tablet, or a hard capsule, it may include lactose, sucrose, starch or a derivative thereof, talc, calcium carbonate, gelatin, stearic acid or a salt thereof. In addition, when the formulation is a soft capsule, it may include vegetable oils, waxes, fats, and semi-solid and liquid polyols. In addition, when the formulation is in the form of a solution or syrup, water, polyol, glycerol, and vegetable oil may be included.

The pharmaceutical composition according to the present invention may further include a preservative, a stabilizer, a wetting agent, an emulsifier, a solubilizing agent, a sweetener, a colorant, an osmotic pressure controller, an antioxidant, and the like, in addition to the carrier described above.

The pharmaceutical composition according to the present invention is administered in a pharmaceutically effective amount. In the present invention, the "pharmaceutically effective amount" is an amount sufficient to treat a disease at a reasonable benefit/risk ratio applicable to medical treatment, and the effective dose level may be determined depending on factors including the patient's disease type, severity, drug activity, drug sensitivity, administration time, administration route, excretion rate, duration of treatment, and drugs taken therewith, and other factors well known in the medical field. The pharmaceutical composition according to the present invention may be administered as an individual therapeutic agent or in combination with other therapeutic agents, may be administered sequentially or simultaneously with conventional therapeutic agents, and may be administered one or multiple times. Taking into consideration all of the above factors, it is important to administer the composition in an amount capable of obtaining the maximum effect using a minimum amount without side effects, which may be easily determined by those skilled in the art.

The pharmaceutical composition of the present invention may be administered to a subject through various routes. The mode of administration may be, for example, oral administration. Administration may be carried out through subcutaneous injection, intravenous injection, intramuscular injection, intrauterine dural injection, or intracerebrovascular injection. The mode of administration of the pharmaceutical composition of the present invention is determined depending on the type of drug as the active ingredient, along with several related factors such as the disease to be treated, the route of administration, the age, gender, and body weight of the patient, and the severity of disease. The pharmaceutical composition and medicament containing the encapsulated probiotics according to the present invention may be used for the treatment or prevention of digestive diseases, and in particular may exhibit the effect of improving the functions of the stomach, colon, and small intestine.

Specifically, it may be used for (i) amelioration, prevention, or treatment of any symptom selected from the group consisting of dyspepsia, loss of appetite, anorexia, overeating, indigestion, stomach bloating due to dyspepsia, constipation, loose stool, diarrhea, and abdominal bloating, (ii) inhibition of abnormal intestinal fermentation, and/or (iii) digestion promotion or enhancement of intestinal function, but the present invention is not limited thereto.

A further aspect of the present invention pertains to the use of the encapsulated probiotics of the present invention for the prevention or treatment of disease.

Still a further aspect of the present invention pertains to a method of treating a disease including administering the encapsulated probiotics of the present invention to a subject.

In the present invention, the disease may be a digestive disease, and the method may be used for (i) amelioration, prevention, or treatment of any symptom selected from the group consisting of dyspepsia, loss of appetite, anorexia, overeating, indigestion, stomach bloating due to dyspepsia, constipation, loose stool, diarrhea, and abdominal bloating, (ii) inhibition of abnormal intestinal fermentation, and/or (iii) digestion promotion or enhancement of intestinal function, but the present invention is not limited thereto.

Yet a further aspect of the present invention pertains to a cosmetic composition including the encapsulated probiotics according to the present invention.

Still yet a further aspect of the present invention pertains to the use of the encapsulated probiotics according to the present invention for the manufacture of a cosmetic composition.

In the present invention, the cosmetic composition may be applied to various functions and uses depending on the efficacy of the encapsulated probiotics, and may be used for, for example, moisturizing the skin, strengthening the skin barrier, soothing the skin, preventing or ameliorating skin damage, or ameliorating the skin barrier, preventing or ameliorating aging, or improving skin condition, but the present invention is not limited thereto.

In the present invention, improving skin condition is used in a generic sense including not only improvement of visible skin condition, but also factors that directly and indirectly affect skin health.

In the present invention, the cosmetic composition may further include a cosmetically acceptable excipient or carrier, and, for example, may further include a variety of known additives depending on the dosage form. For example, any additive selected from the group consisting of a carrier, an emulsifier, a moisturizer, a surfactant, a chelating agent, an antioxidant, a disinfectant, a stabilizer, and combinations thereof may be further included.

In the present invention, examples of the carrier may include, but are not limited to, animal oil, vegetable oil, wax, paraffin, starch, tragacanth, cellulose derivative, polyethylene glycol, silicone, bentonite, silica, talc, zinc oxide, lactose, silica, aluminum hydroxide, calcium silicate, polyamide powder, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, liquid diluents, suspension agents such as ethoxylated isostearyl alcohol, polyoxyethylene sorbitol ester and polyoxyethylene sorbitan ester, microcrystalline cellulose, aluminum metahydroxide, bentonite, agar or tragacanth, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, a lanolin derivative, an ethoxylated glycerol fatty acid ester, and combinations thereof.

In the present invention, examples of the emulsifier may include, but are not limited to, liquid paraffin, cetyl octanoate, stearic acid, and the like.

In the present invention, examples of the moisturizer may include, but are not limited to, glycerin, butylene glycol, propylene glycol, dipropylene glycol, pentylene glycol, hexylene glycol, polyethylene glycol, sorbitol, and the like.

In the present invention, examples of the chelating agent may include, but are not limited to, sodium ethylenediaminetetraacetate (EDTA), α-hydroxy fatty acid, lactoferrin, α-hydroxy acid, citric acid, lactic acid, malic acid, biliverdin, and the like.

In the present invention, examples of the antioxidant may include, but are not limited to, butylhydroxyanisole, dibutylhydroxytoluene, propyl gallate, and the like.

In addition, the cosmetic composition of the present invention may further include at least one active ingredient, in addition to the encapsulated probiotics of the present invention.

In the present invention, the cosmetic composition may be prepared and used in a variety of known formulations according to the intended use and method. For example, the cosmetic composition according to the present invention may take a form such as a lotion, facial lotion, body lotion, nourishing cream, moisture cream, eye cream, essence, cosmetic ointment, spray, gel, pack, sunscreen, makeup base, foundation, powder, cleansing cream, cleansing lotion, cleansing oil, cleansing foam, soap, or body wash, but is not limited thereto.

In the cosmetic composition for skin improvement according to the present invention, when the skin is scalp or hair, the cosmetic composition may take a form such as a hair tonic, hair conditioner, hair essence, hair lotion, hair nutrition lotion, hair shampoo, hair rinse, hair treatment, hair cream, hair nutrition cream, hair moisture cream, hair massage cream, hair wax, hair aerosol, hair pack, hair nutrition pack, hair soap, hair cleansing foam, hair oil, hair drying agent, hair preservative, hair dyeing agent, hair wave agent, hair bleaching agent, hair gel, hair glaze, hairdressing, hair lacquer, hair moisturizer, hair mousse or hair spray, but is not limited thereto.

In the present invention, the encapsulated probiotics as the active ingredient of the cosmetic composition and the additive may be included in cosmetically acceptable amounts, and are preferably included within the scope of regulations set by each country, for example, within a range that does not exceed the maximum amount set forth in the ^{┌}Technical Guidelines for Cosmetic Safety Assessment_{┘} set by the Chinese government.

In the present invention, the cosmetic composition may be included in functional cosmetics. The standard for the functional cosmetics may be in accordance with the ^{┌}Functional Cosmetics Standards and Test Methods_{┘} notified by the Ministry of Food and Drug Safety in Korea, and for example, the cosmetic composition may have functions such as whitening, wrinkle reduction, hair color change, UV protection, acne alleviation, hair loss reduction, etc., but the present invention is not limited thereto.

In the present invention, the cosmetic composition may be included in drug-containing cosmetics or general cosmetics set forth in ^{┌}Standards for Cosmetics containing Medical or Toxic Drugs_{┘} set by the Taiwanese government.

A better understanding of the present invention may be obtained through the following examples. These examples are merely set forth to illustrate the present invention, and are not to be construed as limiting the scope of the present invention, as will be apparent to those skilled in the art.

### Examples

### Experimental Example 1. Confirmation of properties of probiotic capsules obtained through method of producing encapsulated probiotics according to the present invention

Control and Examples used in Experimental Example 1 are shown in Table 2 below.

**[Table 2]**

| Conditions | Addition to medium | Cell treatment conditions |
|---|---|---|
| Control 1 | Calcium carbonate | - |
| Control 2 | Calcium carbonate | Cryoprotectant |
| Example 1 | Calcium carbonate + sodium alginate | - |
| Example 2 | Calcium carbonate + sodium alginate + starch | - |

### Control 1

4 L of a probiotic culture medium (containing 30 g/L of glucose (Daesang), 10 g/L of a yeast extract (Biospringer), 15 g/L of hydrolyzed soy protein (Tatua), and 0.1 g/L of magnesium sulfate (Kali)) supplemented with 3 g/L of calcium carbonate was prepared in a 7.5 L jar fermentor and sterilized at 121°C for 25 minutes. *Lactobacillus plantarum* IDCC 3501 (KCTC accession number: 13586BP), *Lactobacillus reuteri* IDCC 3701, or *Enterococcus faecium* IDCC 2102 was inoculated into the prepared medium so that the initial viable cell count of the strain was about 1×10⁸ CFU/mL, followed by culture at 37°C at 150 rpm for 16 hours. After termination of culture, the culture broth was cooled to 20°C and then centrifuged to collect cells, which were then freeze-dried.

### Control 2

4 L of a probiotic culture medium (containing 30 g/L of glucose (Daesang), 10 g/L of a yeast extract (Biospringer), 15 g/L of hydrolyzed soy protein (Tatua), and 0.1 g/L of magnesium sulfate (Kali)) supplemented with 3 g/L of calcium carbonate was prepared in a 7.5 L jar fermentor and sterilized at 121°C for 25 minutes. *Lactobacillus plantarum* IDCC 3501 (KCTC accession number: 13586BP), *Lactobacillus reuteri* IDCC 3701, or *Enterococcus faecium* IDCC 2102 was inoculated into the prepared medium so that the initial viable cell count of the strain was about 1×10⁸ CFU/mL, followed by culture at 37°C at 150 rpm for 16 hours. After termination of culture, the culture broth was cooled to 20°C and then centrifuged to collect cells, which were then mixed with a cryoprotectant (100 g/L of maltodextrin + 50 g/L of fructooligosaccharide) in the same amount (v/w) with the cell solution and freeze-dried.

### Example 1

4 L of a probiotic culture medium (containing 30 g/L of glucose (Daesang), 10 g/L of a yeast extract (Biospringer), 15 g/L of hydrolyzed soy protein (Tatua), and 0.1 g/L of magnesium sulfate (Kali)) supplemented with 3 g/L of calcium carbonate and 1 g/L of sodium alginate was prepared in a 7.5 L jar fermentor and sterilized at 121°C for 25 minutes. *Lactobacillus plantarum* IDCC 3501 (KCTC accession number: 13586BP), *Lactobacillus reuteri* IDCC 3701, or *Enterococcus faecium* IDCC 2102 was inoculated into the prepared medium so that the initial viable cell count of the strain was about 1×10⁸ CFU/mL, followed by culture at 37°C at 150 rpm for 16 hours. After termination of culture, the culture broth was cooled to 20°C and then centrifuged at 6,700 g and 20°C for 15 minutes to collect cells, which were then freeze-dried.

### Example 2

4 L of a probiotic culture medium (containing 30 g/L of glucose (Daesang), 10 g/L of a yeast extract (Biospringer), 15 g/L of hydrolyzed soy protein (Tatua), and 0.1 g/L of magnesium sulfate (Kali)) supplemented with 3 g/L of calcium carbonate, 1 g/L of sodium alginate, and 5 g/L of corn starch was prepared in a 7.5 L jar fermentor and sterilized at 121°C for 25 minutes. *Lactobacillus plantarum* IDCC 3501 (KCTC accession number: 13586BP), *Lactobacillus reuteri* IDCC 3701, or *Enterococcus faecium* IDCC 2102 was inoculated into the prepared medium so that the initial viable cell count of the strain was about 1×10⁸ CFU/mL, followed by culture at 37°C at 150 rpm for 16 hours. After termination of culture, the culture broth was cooled to 20°C and then centrifuged to collect cells, which were then freeze-dried.

### Experimental Example 1-1. Evaluation of freeze-drying viability

The freeze-dried raw material was pulverized using a sieve having a mesh size of 425 um, weighed, and mixed with maltodextrin in an amount corresponding to half the weight of the bulk powder. In order to measure the viable cell count in the culture broth and the raw material for production, serial dilution with a buffer solution (phosphate-citrate buffer, Sigma-Aldrich, P4809), spreading on an MRS agar medium, and then culture at 37°C for 24 hours were performed, after which the resulting colonies were counted. The freeze-drying viability was represented as a percentage of the ratio of the total viable cell count in the raw material relative to the total viable cell count in the culture broth.

The results of analyzing the viable cell count in the freeze-dried powder of each strain are shown in Table 3 below. The viability relative to the viable cell count in the culture broth of the IDCC 3501 strain was 23.5% and 48.8% in Control 1 and 2, respectively, but was 60.6% and 83.8% in Examples 1 and 2, respectively, indicating high freeze-drying viability in the raw material on which encapsulation was carried out. In particular, Example 2, in which both the encapsulant and the encapsulation enhancer were used, showed viability 3.7 times as high as that of Control 1, in which the cells were not additionally treated, and 1.8 times as high as that of Control 2.

Based on the results of analyzing the viable cell count in the freeze-dried powder of the IDCC 3701 and IDCC 2102 strains, it was confirmed that the viability after freeze-drying relative to the culture broth was remarkably increased, as in the IDCC 3501 strain. This indicated that the spontaneously formed encapsulant and encapsulation enhancer can effectively function as a cryoprotectant without the use of an additional cryoprotectant.

**[Table 3]**

| Conditions | Viability after freeze-drying of strain relative to culture broth (%) | | |
|---|---|---|---|
| | IDCC 3501 | IDCC 3701 | IDCC 2102 |
| Control 1 | 23.5 | 34.1 | 57.3 |
| Control 2 | 48.8 | 46.4 | 82.0 |
| Example 1 | 60.6 | 64.6 | 91.3 |
| Example 2 | 83.8 | 81.3 | 94.8 |

### Experimental Example 1-2. Evaluation of heat tolerance

Probiotic capsules undergo a variety of conditions that are lethal when processed in various intake forms, and in particular, when compressed into tablets, they are momentarily exposed to high heat and pressure. In order to confirm the effect of protecting viable cells through encapsulation at high temperatures outside the range within which strain growth is possible, 0.1 g of the viable cell powder for each of the freeze-dried strains was suspended in 10 mL of a buffer solution and then heated in a water bath at 60°C for 1 hour, after which the viability thereof was measured. The heat-treated probiotic suspension was serially diluted, spread on an MRS agar medium, and then cultured at 37°C for 24 hours, and the resulting colonies were counted. The heat tolerance result was represented as a D value, which is the time (min) required to lower the viable cell count to 1/10 of the original count. Here, the D value is obtained from the time required to lower the viable cell count to 1/10 of the original count, and thus, the higher the numerical value, the higher the heat tolerance at 60°C. Based on the results of comparison of the D values, it was confirmed that all strains exhibited much higher heat tolerance in Examples 1 and 2 than in Control 1 and 2 (Table 4 below).

**[Table 4]**

| Conditions | Heat tolerance (1 h; D value (min)) | | |
|---|---|---|---|
| | IDCC3501 | IDCC 3701 | IDCC 2102 |
| Control 1 | 11.7 | 11.1 | 13.9 |
| Control 2 | 14.2 | 12.6 | 14.3 |
| Example 1 | 15.3 | 14.6 | 16.4 |
| Example 2 | 15.5 | 15.4 | 16.3 |

### Experimental Example 1-3. Survival rate in simulated gastrointestinal solution

Probiotics ingested into the human body are exposed to an environment in which it is difficult to survive until they reach the intestine in a viable state. Gastric acid and bile acid secreted by the duodenum are typical examples. Therefore, in order to confirm the ingestion stability of viable probiotics, the survival rate in a solution simulating the gastrointestinal tract conditions when food was ingested was measured.

In order to prepare a simulated gastric solution, 55 g/L of an MRS medium was dissolved in distilled water, adjusted to a pH of 3.0, and then sterilized at 121°C for 15 minutes to make an acidic MRS medium. 0.1 g of the viable cell powder of each freeze-dried strain was suspended in 10 mL of the MRS medium and cultured for 1 hour at 37°C with stirring at 100 rpm. 1 mL of the suspension was serially diluted, spread on an MRS agar medium, and then cultured at 37°C for 24 hours, and the resulting colonies were counted.

In order to prepare a simulated intestinal solution, bile (Oxgall, Sigma-Aldrich) was dissolved at a concentration of 4 g/L in distilled water and then adjusted to a pH of 7.4. 90 mL of the simulated intestinal solution was added to the suspension for the simulated gastric solution, cultured for 2 hours at 37°C with stirring at 100 rpm, and sampled at 1-hour intervals, followed by serial dilution, spreading on an MRS agar medium, and culture at 37°C for 24 hours, after which the resulting colonies were counted.

When the probiotic powder was treated in the simulated gastrointestinal solution, the IDCC 3501 strain exhibited survival rates of 0.3% and 15.0% in Control 1 and 2, respectively, and survival rates of 24.9% and 59.8% in Examples 1 and 2, respectively. In particular, Example 2 exhibited acid resistance of 90% or more in the simulated gastric solution, and finally, after 3 hours of treatment, the survival rate thereof was 721 times as high as that of Control 1, and 7.2 times as high as that of Control 2 (Table 5 below).

The IDCC 3701 and IDCC 2102 strains also exhibited survival rates increased by at least 1.6 times to a maximum of 60 times in Examples 1 and 2 compared to Control 1 and 2 (Table 6 below).

This means that, in the freeze-dried probiotic formulation of the present invention, the spontaneously formed encapsulant and encapsulation enhancer can effectively protect cells in an actual gastrointestinal tract.

**[Table 5]**

| Conditions | Viable cell count of *Lactobacillus plantarum* IDCC 3501 strain (CFU/g) | | | | Final survival rate (%) |
|---|---|---|---|---|---|
| | 0 h (initial value) | 1 h (simulated gastric solution) | 2 h (simulated intestinal solution) | 3 h (simulated intestinal solution) | |
| Control 1 | 1.10E+11 | 1.14E+09 | 3.29E+08 | 3.40E+08 | 0.3 |
| Control 2 | 2.28E+11 | 4.13E+10 | 3.57E+10 | 3.41E+10 | 15.0 |
| Example 1 | 2.84E+11 | 1.47E+11 | 7.69E+10 | 7.08E+10 | 24.9 |
| Example 2 | 4.10E+11 | 3.95E+11 | 2.37E+11 | 2.45E+11 | 59.8 |

**[Table 6]**

| Conditions | Survival rate after 3 hours of treatment in simulated gastrointestinal solution (%) | | |
|---|---|---|---|
| | IDCC 3501 | IDCC 3701 | IDCC 2102 |
| Control 1 | 0.3 | 1.2 | 8.3 |
| Control 2 | 15.0 | 19.7 | 33.9 |
| Example 1 | 24.9 | 32.6 | 56.8 |
| Example 2 | 59.8 | 61.8 | 83.0 |

### Experimental Example 1-4. Shelf stability

During distribution and storage of dried probiotics, viable cell activity gradually decreases due to factors such as temperature, oxygen, moisture, and the like. A physical barrier such as encapsulation is capable of contributing to improving shelf stability by protecting the probiotic cells from such lethal factors. Although it is possible to confer protection against oxygen and moisture due to the use of antioxidants and deoxidizers or through moisture-proofing treatment in the raw material mixing and packaging steps, ability of the raw material itself to withstand the temperature to which it is exposed is required. Therefore, the freeze-dried probiotic powder was mixed with dry corn starch as an excipient in an amount corresponding to 1 to 10 times the amount of the probiotic powder in order to suppress the hygroscopicity of raw material, and then separately packaged in an aluminum pouch to prevent contact with oxygen and moisture. The packaged raw material was stored for 7 days under harsh conditions of 40°C and 50°C, and then short-term viability was measured, and long-term viability was measured after storage at 25°C and 30°C for 90 days. The stored raw material was serially diluted with a buffer solution, spread on an MRS agar medium, and then cultured at 37°C for 24 hours, and the resulting colonies were counted.

Based on the results of measurement of viability after storing the probiotic powder for 7 days under harsh temperature conditions, the viability of the IDCC 3501 strain at 40°C was 1.1% and 14.7% in Control 1 and 2, respectively, and 40.1% and 87.2% in Examples 1 and 2, respectively. Also, the viability thereof at 50°C was 0.03% and 0.2% in Control 1 and 2, respectively, and 5.4% and 14.3% in Examples 1 and 2, respectively. Consequently, the raw material on which the encapsulation process was carried out showed much higher short-term shelf stability than Control 1, in which the cells were simply dried without any post-treatment, and Control 2, which was dried using a cryoprotectant. In addition, the stability under harsh conditions of Example 2, in which both the encapsulant and the encapsulation enhancer were cultured, was at least twice as high as that of Example 1, in which the encapsulant alone was cultured (Tables 7 and 8 below).

The IDCC 3701 and IDCC 2102 strains also exhibited vastly superior stability under harsh conditions compared to Control, indicating that the freeze-dried formulation of lactic acid bacteria produced through the technique for spontaneously forming the matrix capsule protective film according to the present invention has very high shelf stability.

**[Table 7]**

| Conditions | Viability at 40°C for 7 days (%) | | | Viability at 50°C for 7 days (%) | | |
|---|---|---|---|---|---|---|
| | IDCC 3501 | IDCC 3701 | IDCC 2102 | IDCC 3501 | IDCC 3701 | IDCC 2102 |
| Control 1 | 3.8 | 4.8 | 31.3 | 0.03 | 0.07 | 3.8 |
| Control 2 | 14.7 | 16.3 | 83.7 | 0.9 | 3.0 | 11.2 |
| Example 1 | 40.1 | 51.3 | 97.5 | 5.4 | 7.9 | 26.6 |
| Example 2 | 87.2 | 86.2 | 98.6 | 14.3 | 14.2 | 29.3 |

**[Table 8]**

| Conditions | Viability at 25°C for 90 days (%) | | | Viability at 30°C for 90 days (%) | | |
|---|---|---|---|---|---|---|
| | IDCC 3501 | IDCC 3701 | IDCC 2102 | IDCC 3501 | IDCC 3701 | IDCC 2102 |
| Control 1 | 3.8 | 4.8 | 31.3 | 0.03 | 0.07 | 3.8 |
| Control 2 | 14.7 | 16.3 | 83.7 | 0.9 | 3.0 | 11.2 |
| Example 1 | 40.1 | 51.3 | 97.5 | 5.4 | 7.9 | 26.6 |
| Example 2 | 87.2 | 86.2 | 98.6 | 14.3 | 14.2 | 29.3 |

Based on the results of measurement of viability after storing the probiotic powder at 25°C and 30°C for 90 days, the viability of the IDCC 3501 strain at 25°C was 1.9% and 17.8% in Control 1 and 2, respectively, and 56.0% and 95.7% in Examples 1 and 2, respectively, and additionally, the viability thereof at 30°C was 0.09% and 8.3% in Control 1 and 2, respectively, and 35.0% and 86.5% in Examples 1 and 2, respectively. Similar to the above results for harsh conditions, shelf stability was the highest for Example 2, followed by Example 1, Control 2, and Control 1, and in particular, Example 2, which is a preferred embodiment of the present invention, showed high stability of 90% or more at 25°C.

The IDCC 3701 and IDCC 2102 strains also exhibited vastly superior stability under harsh conditions compared to Control, indicating that the freeze-dried formulation of lactic acid bacteria produced through the technique for spontaneously forming the matrix capsule protective film according to the present invention has very high shelf stability not only upon short-term storage but also upon long-term storage for 90 days or longer (Tables 7 and 8).

### Experimental Example 2. Comparison of properties of probiotic capsules produced through encapsulation method of the present invention or conventional alginate encapsulation method

Control (typical method of producing calcium alginate raw material) and Example used in Experimental Example 2 were as follows.

### Control 1

4 L of a probiotic culture medium (containing 30 g/L of glucose (Daesang), 10 g/L of a yeast extract (Biospringer), 15 g/L of hydrolyzed soy protein (Tatua), and 0.1 g/L of magnesium sulfate (Kali)) supplemented with 3 g/L of calcium carbonate was prepared in a 7.5 L jar fermentor and sterilized at 121°C for 25 minutes. *Lactobacillus plantarum* IDCC 3501 (KCTC accession number: 13586BP) was inoculated into the prepared medium so that the initial viable cell count thereof was about 1×10⁸ CFU/mL, followed by culture at 37°C at 150 rpm for 16 hours. After termination of culture, the culture broth was cooled to 20°C and then centrifuged to collect 400 mL of a cell solution. The cell solution thus centrifuged was mixed with a suspension obtained by adding 10 mL of distilled water with 0.4 g of sodium alginate and 2 g of corn starch, homogenized, and then added dropwise with a 1% calcium chloride aqueous solution to thus prepare the same in a bead form, after which freeze-drying was performed and the dried bulk powder was collected.

### Control 2

The centrifuged cell solution prepared in the same manner as in Control 1 was added with a hydrogel obtained by adding 0.4 g of sodium alginate, 2 g of corn starch, and 1.2 g of calcium carbonate to 100 mL of distilled water and adding a 10% lactic acid solution at a rate of 0.1 mL/sec up to a pH of 4.00 with stirring at 200 rpm, homogenized, and then freeze-dried. The freeze-dried cells were pulverized using a sieve having a mesh size of 425 µm, weighed, and mixed with maltodextrin in an amount corresponding to half the weight of the bulk powder.

### Example 1

4 L of a probiotic culture medium (containing 30 g/L of glucose (Daesang), 10 g/L of a yeast extract (Biospringer), 15 g/L of hydrolyzed soy protein (Tatua), and 0.1 g/L of magnesium sulfate (Kali)) supplemented with 3 g/L of calcium carbonate, 1 g/L of sodium alginate, and 5 g/L of corn starch was prepared in a 7.5 L jar fermentor and sterilized at 121°C for 25 minutes. *Lactobacillus plantarum* IDCC 3501 (KCTC accession number: 13586BP) was inoculated into the prepared medium so that the initial viable cell count thereof was about 1×10⁸ CFU/mL, followed by culture at 37°C at 150 rpm for 16 hours. After termination of culture, the culture broth was cooled to 20°C and then centrifuged to collect cells, which were then freeze-dried.

### Experimental Example 2-1. Evaluation of freeze-drying viability

In order to measure the viable cell count in the culture broth and the raw material for production in Control and Example, freeze-drying viability was evaluated in the same manner as in Experimental Example 1-1.

Based on the results of analyzing the viable cell count in the freeze-dried powder and confirming the viability relative to the viable cell count in the culture broth, freeze-drying viability was 68.7% and 43.1% in Control 1 and 2, respectively, but was 84.1% in Example 1, which was regarded as relatively high. This indicated that forming the encapsulant in the culture step can more effectively serve as a cryoprotectant than post-treatment on the cells (Table 9 below).

**[Table 9]**

| Conditions | Viable cell count in raw material | Viability relative to culture broth (%) |
|---|---|---|
| Control 1 | 3.21E+11 | 68.7 |
| Control 2 | 2.01E+11 | 43.1 |
| Example 1 | 4.19E+11 | 84.1 |

### Experimental Example 2-2. Evaluation of heat tolerance

Based on the results of evaluating the heat tolerance of the viable cell powder of Control and Example in the same manner as in Experimental Example 1-2, the D values of Control 1 and 2 were 14.8 minutes and 14.2 minutes, respectively, but the D value of Example 1 was 15.7 minutes, which was regarded as relatively high. Therefore, it was confirmed that the raw material produced through the encapsulation culture process of the present invention exhibited remarkably high heat tolerance (Table 10 below).

**[Table 10]**

| Conditions | Heat tolerance (1 h; D value (min)) |
|---|---|
| | IDCC 3501 |
| Control 1 | 14.8 |
| Control 2 | 14.2 |
| Example 1 | 15.7 |

### Experimental Example 2-3: Survival rate in simulated gastrointestinal solution

Each produced raw material was measured for the survival rate and disintegration rate in the simulated gastrointestinal solution. The survival rate in the simulated gastrointestinal solution was evaluated in the same manner as in Experimental Example 1-3, and upon analysis after 2 hours of treatment in the simulated intestinal solution, additional analysis using saline (0.9% sodium chloride) as dilution water was performed, and the disintegration rate in the simulated gastrointestinal solution was determined based on the ratio of the same to analysis results obtained by completely dissolving alginate in a buffer solution (citrate-phosphate buffer).

The difference in the final survival rate between Example 1 and Control 1 was about 2.1%, which indicates similarity therebetween, but the disintegration rate of Example 1 was at least four times higher. These results suggest that, when encapsulated in the bead form, as in Control 1, the ability to protect probiotics from the simulated gastrointestinal solution is excellent, but it is difficult for probiotics to effectively disintegrate and proliferate in the gastrointestinal tract as in Example 1. Control 2 showed a disintegration rate similar to that of Example 1, but the survival rate was lower than that of Example 1, and thus the ability to provide protection against the simulated gastrointestinal solution was insufficient (Tables 11 and 12 below). Therefore, it was confirmed that the encapsulation method of the present invention exhibited excellent disintegration and stability in an actual gastrointestinal tract compared to the conventional alginate encapsulation method.

**[Table 11]**

| Conditions | Viable cell count (CFU/g) | | | | 3 h survival rate (%) |
|---|---|---|---|---|---|
| | 0 h (initial value) | 1 h (simulated gastric solution) | 2 h (simulated intestinal solution) | 3 h (simulated intestinal solution) | |
| Control 1 | 3.21E+11 | 2.92E+11 | 1.83E+11 | 1.80E+11 | 56.1 |
| Control 2 | 2.01E+11 | 1.32E+11 | 6.92E+11 | 6.77E+11 | 33.6 |
| Example 1 | 4.19E+11 | 3.96E+11 | 2.46E+11 | 2.44E+11 | 58.2 |

**[Table 12]**

| Conditions | Viable cell count (CFU/g) | Disintegration rate (%) |
|---|---|---|
| | 3 h (simulated intestinal solution, diluted with saline) | |
| Control 1 | 4.09E+10 | 22.8 |
| Control 2 | 6.53E+10 | 96.5 |
| Example 1 | 2.39E+11 | 97.8 |

### Experimental Example 2-4. Shelf stability

Each produced raw material was packaged in the same manner as in Experimental Example 1-4, and the packaged raw material was stored for 7 days under harsh conditions of 40°C and 50°C, after which short-term viability was measured, and long-term viability was measured after storage at 25°C and 30°C for 90 days. The stored raw material was serially diluted with a buffer solution, spread on an MRS agar medium, and then cultured at 37°C for 24 hours, and the resulting colonies were counted.

Based on the results of measurement of viability after storage of the probiotic powder for 7 days under harsh temperature conditions, the viability thereof at 40°C was 72.5% and 36.1% in Control 1 and 2, respectively, but was 88.1% in Example 1, which was regarded as relatively high. Also, the viability thereof at 50°C was 10.2% and 4.3% in Control 1 and 2, respectively, but was 15.4% in Example 1, which was high compared to Control. Consequently, the raw material of Example 1 in which the encapsulation process was carried out in the culture step showed high short-term shelf stability compared to the raw materials of Control to which alginate was applied in the post-treatment step (Table 13 below).

Based on the results of measurement of viability depending on the temperature after storing the probiotic powder for 90 days, the viability thereof at 25°C was 76.3% and 49.1% in Control 1 and 2, respectively, but was 97.2% in Example 1, which was regarded as relatively high, and the viability thereof at 30°C was 58.2% and 31.0% in Control 1 and 2, respectively, but was 86.5% in Example 1, which was also relatively high. Like the short-term shelf stability results, Example 1 exhibited high long-term shelf stability compared to Control 1 and 2, and in particular, showed very high viability of 95% or more at 25°C (Table 14 below).

**[Table 13]**

| Conditions | Viability at 40°C for 7 days (%) | | Viability at 50°C for 7 days (%) | |
|---|---|---|---|---|
| | Viable cell count (CFU/g) | Viability (%) | Viable cell count (CFU/g) | Viability ) |
| Control 1 | 2.32E+11 | 72.5 | 3.26E+10 | 10.2 |
| Control 2 | 7.27E+10 | 36.1 | 8.73E+9 | 4.3 |
| Example 1 | 3.69E+11 | 88.1 | 6.46E+10 | 15.4 |

**[Table 14]**

| Conditions | Viability at 25°C for 90 days (%) | | Viability at 30°C for 7 days (%) | |
|---|---|---|---|---|
| | Viable cell count (CFU/g) | Viability (%) | Viable cell count (CFU/g) | Viability (%) |
| Control 1 | 2.45E+11 | 76.3 | 1.87E+11 | 58.2 |
| Control 2 | 9.88E+10 | 49.1 | 6.23E+10 | 31.0 |
| Example 1 | 4.07E+11 | 97.2 | 3.63E+11 | 86.5 |

### Experimental Example 2-5. Analysis of particle size of encapsulant

The particle sizes were compared between Example 1, in which the encapsulant was spontaneously formed in the culture step, and Control 2, in which the encapsulant was artificially formed *in vitro.* The cell solution obtained in the centrifugation step in Example 1 was diluted with saline and then filtered three times with cellulose filter paper (1004047, Whatman) to collect pellets, which were then freeze-dried. The particle size was analyzed using a particle size analyzer (LS-I3-320, Beckman Coulter) by dissolving the dried encapsulant of each of Example 1 and Control 2 in saline.

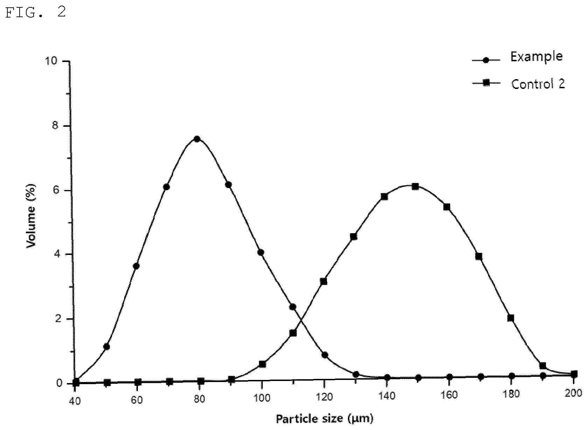

Based on the results of analysis of the particle size of the dried encapsulant, the average particle size of the encapsulant was 82.45 um in Example 1 and 146.09 um in Control 2, indicating that the encapsulant of Example 1 exhibited a fine particle size (FIG. 2). Therefore, it was confirmed that smaller cell gaps could be filled during encapsulation of cells in Example 1, in which the encapsulant was spontaneously formed in the culture step, thereby contributing to the improvement of stability.

Based on the above results of Experimental Examples, compared to the processes of Control 1, using the alginate encapsulation process in the bead form, and Control 2, in which alginate was separately prepared and then mixed with the cell solution using the same principle as in Example, the method of preparing the encapsulant according to the present invention, in which the alginate encapsulant was spontaneously formed and mixed with the cell solution, exhibited vastly superior freeze-drying viability, heat tolerance, survival rate in the simulated gastrointestinal solution, and short-term/long-term shelf stability. In particular, it was confirmed that the encapsulant prepared through the method of the present invention is very fine particles and thus can exert a protective effect from various factors, which adversely affect the survival of probiotics, by filling the gaps between the cells therewith. Therefore, it was confirmed that, when the probiotic strain is encapsulated through the method of the present invention, high ability to protect cells is exhibited compared to the typical alginate encapsulation process.

### Experimental Example 3. Structural properties of probiotic capsules

The encapsulation raw material of *Lactobacillus plantarum* IDCC 3501 (KCTC accession number: 13586BP) was observed using an SEM. Dry cells on which the encapsulation process of the present invention was not carried out, dry cells to which only the encapsulant was applied (the same as Example 1 of Experimental Example 1), and dry cells to which both the encapsulant and the encapsulation enhancer were applied (the same as Example 2 of Experimental Example 1) were observed at 25,000X magnification using an FE-SEM (S-4700, Hitachi).

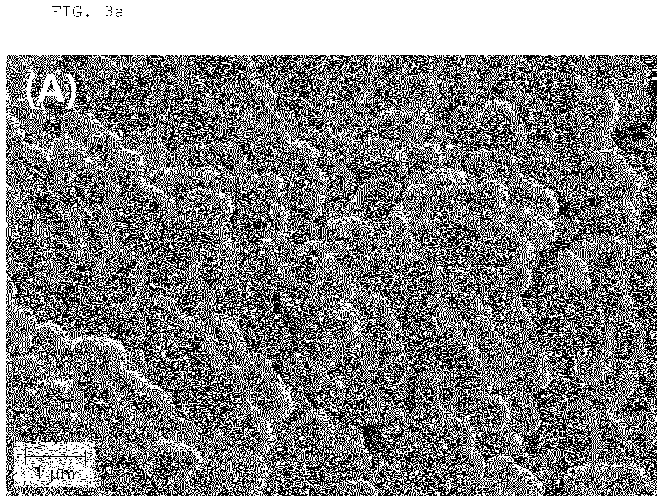

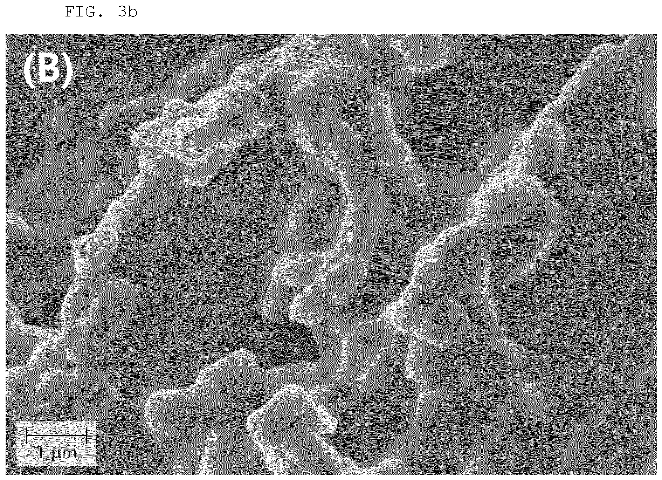

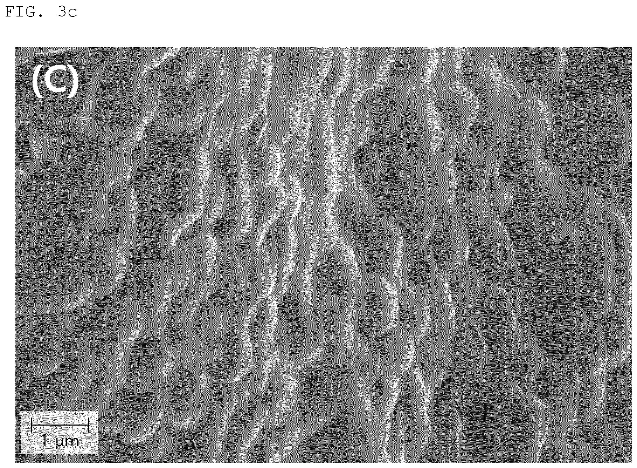

In the dry cells (A) on which encapsulation was not carried out, there was a significant gap between cells, but in the dry cells (B) to which only the encapsulant was applied and in the dry cells (C) to which both the encapsulant and the encapsulation enhancer were applied, the cells were surrounded by the encapsulant and the gaps therebetween were filled. Also, when observing the three-dimensional structure of cell colonies, the dry cells to which the encapsulation enhancer was applied had a relatively small surface area compared to the dry cells to which only the encapsulant was applied, indicating that a structure that is relatively less exposed to the environment that threatens the survival of probiotics (FIG. 3) was formed.

Therefore, it was confirmed that the probiotic capsules produced through the encapsulation method according to the present invention were structurally very stable.

Although specific embodiments of the present invention have been disclosed in detail above, it will be obvious to those skilled in the art that the description is merely of preferable exemplary embodiments and is not to be construed as limiting the scope of the present invention. Therefore, the substantial scope of the present invention will be defined by the appended claims and equivalents thereof.

## Claims

1. A method of producing encapsulated probiotics with an alginate hydrogel, comprising:
(a) spontaneously forming an alginate hydrogel simultaneously with proliferation of probiotics by culturing probiotics in a medium containing an alginate and a salt that forms a hydrogel by binding to alginic acid; and
(b) recovering the probiotics encapsulated by the spontaneously formed alginate hydrogel.

2. The method according to claim 1, wherein the probiotics of the encapsulated probiotics are entrapped within the alginate hydrogel.

3. The method according to claim 1, wherein the encapsulated probiotics are of a matrix type.

4. The method according to claim 1, wherein, in step (a), an acid generated by culturing the probiotics dissociates a cation from the salt that forms a hydrogel by binding to alginic acid, and the dissociated cation and the alginic acid are coupled with each other to thus spontaneously form the alginate hydrogel.

5. The method according to claim 1, wherein the alginate has solubility in water of 0.1 g/L or more.

6. The method according to claim 5, wherein the alginate is selected from the group consisting of sodium alginate, potassium alginate, magnesium alginate, calcium alginate, lithium alginate, and ammonium alginate.

7. The method according to claim 1, wherein the salt that forms a hydrogel by binding to alginic acid has solubility in water of 0.1 g/L or less.

8. The method according to claim 7, wherein the salt that forms a hydrogel by binding to alginic acid is a carbonate or nitrate of a divalent cation.

9. The method according to claim 8, wherein the carbonate is selected from the group consisting of calcium carbonate, barium carbonate, manganese carbonate, copper carbonate, zinc carbonate, lead carbonate, cadmium carbonate, cobalt carbonate, nickel carbonate, and strontium carbonate.

10. The method according to claim 8, wherein the nitrate is selected from the group consisting of calcium nitrate, barium nitrate, manganese nitrate, copper nitrate, zinc nitrate, lead nitrate, cadmium nitrate, cobalt nitrate, nickel nitrate, and strontium nitrate.

11. The method according to claim 1, wherein the alginate is contained in an amount of 0.1 to 40 g/L in the medium, and the salt that forms a hydrogel by binding to alginic acid is contained in an amount of 0.5 to 10 g/L in the medium.

12. The method according to claim 1, wherein the medium in step (a) further contains an encapsulation enhancer.

13. The method according to claim 12, wherein the encapsulation enhancer is selected from the group consisting of starch, crystalline cellulose, chitosan, carboxymethyl cellulose (CMC), and skim milk powder.

14. The method according to claim 13, wherein the encapsulation enhancer is contained in an amount of 1 to 20 g/L in the medium.

15. The method according to claim 1, wherein the probiotics are selected from the group consisting of *Lactobacillus* sp., *Bifidobacterium* sp., *Streptococcus* sp., *Lactococcus* sp., *Enterococcus* sp., *Leuconostoc* sp., *Pediococcus* sp., and *Weissella* sp.

16. The method according to claim 1, further comprising (c) freeze-drying the recovered probiotics encapsulated by the alginate hydrogel.

17. Encapsulated probiotics produced through the method according to any one of claims 1 to 16.

18. The encapsulated probiotics according to claim 17, wherein probiotics are entrapped within an alginate hydrogel.

19. A food or a functional health food comprising the encapsulated probiotics according to claim 17.

20. A pharmaceutical composition or a medicament comprising the encapsulated probiotics according to claim 17.

21. The pharmaceutical composition or the medicament according to claim 20, which is used for treatment or prevention of a digestive disease.

22. The pharmaceutical composition or the medicament according to claim 20, which is used for:
(i) amelioration, prevention, or treatment of any symptom selected from the group consisting of dyspepsia, loss of appetite, anorexia, overeating, indigestion, stomach bloating due to dyspepsia, constipation, loose stool, diarrhea, and abdominal bloating;
(ii) inhibition of abnormal intestinal fermentation; and/or
(iii) digestion promotion or enhancement of intestinal function.

23. A cosmetic composition comprising the encapsulated probiotics according to claim 17.
